# EUROPEAN PATENT SPECIFICATION

(11) **EP 3 946 295 B1**
(45) Date of publication and mention of the grant of the patent: **05.08.2026**
(21) Application number: 20712594.9
(22) Date of filing: 25.03.2020
(51) Int. Cl.: A61K 31/135, A61K 39/00, A61K 45/06, A61P 35/00, A61K 39/395

(54) **COMBINATIONS OF IADADEMSTAT FOR CANCER THERAPY**
KOMBINATIONEN VON IADADEMSTAT FÜR DIE KREBSTHERAPIE
COMBINAISONS D'IADADEMSTAT POUR LA THÉRAPIE DU CANCER

(30) Priority: 25.03.2019 EP 19382206
(43) Date of publication of application: 09.02.2022
(73) Proprietor: Oryzon Genomics, S.A., 28014 Madrid (ES)
(72) Inventor: CICERI, Filippo, 08940 Cornellà de Llobregat (ES)
(74) Representative: Vossius & Partner Patentanwälte Rechtsanwälte mbB
(86) International application number: PCT/EP2020/058362
(87) International publication number: WO 2020/193631

(56) References cited:
- WO-A1-2018/083138
- WO-A1-2019/083971
- JUNZO HAMANISHI ET AL: "PD-1/PD-L1 blockade in cancer treatment: perspectives and issues", INTERNATIONAL JOURNAL OF CLINICAL ONCOLOGY, vol. 21, no. 3, 22 February 2016 (2016-02-22), GB, pages 462 - 473, XP055535295, ISSN: 1341-9625, DOI: 10.1007/s10147-016-0959-z
- WANQIANG SHENG ET AL: "LSD1 Ablation Stimulates Anti-tumor Immunity and Enables Checkpoint Blockade", CELL, vol. 174, no. 3, 1 July 2018 (2018-07-01), AMSTERDAM, NL, pages 549 - 563, XP055616669, ISSN: 0092-8674, DOI: 10.1016/j.cell.2018.05.052
- TAMARA MAES, MASCARÓ CRISTINA, SACILOTTO NATALIA, LUFINO MICHELE MP, CICERI FILIPPO: "Targeting KDM1A with iadademstat in combination with immunotherapy in an in vivo model of melanoma", vol. 37, 26 May 2019 (2019-05-26), pages e14248, XP055699502, Retrieved from the Internet <URL:https://ascopubs.org/doi/10.1200/JCO.2019.37.15_suppl.e14248> DOI: 10.1200/JCO.2019.37.15_suppl.e14248

## Description

### FIELD

The present invention relates to combinations of iadademstat for cancer therapy, in particular combinations with immune checkpoint inhibitors as defined in the appended claims.

### BACKGROUND

Cancer immunotherapy, also referred to as immuno-oncology, is the artificial stimulation of the immune system to treat cancer. Checkpoint inhibitor therapy is a form of cancer immunotherapy that targets immune checkpoints, key regulators of the immune system that stimulate or inhibit its actions, which tumors can use to protect themselves from attacks by the immune system. Checkpoint therapy can block inhibitory checkpoints, restoring immune system function. In particular, PD(L)1 inhibitors are a group of immune checkpoint inhibitors that act to inhibit the association of programmed cell death protein 1 (PD-1, also called PDCD1 or CD279) to its ligands, programmed death-ligand 1 (PD-L1, also known as CD274) and programmed death-ligand 2 (PD-L2, also known as CD273). The interaction of these cell surface proteins is involved in the suppression of the immune system and occurs following infection to limit the killing of bystander host cells and prevent autoimmune disease, but also in different types of cancer.

By recruiting the immune system against cancer cells, PD(L)1 inhibitor therapy holds promise to achieve long-lasting responses in several malignant conditions, and several PD-1 inhibitors and PD-L1 inhibitors have already been approved as a treatment for several types of cancer. However, a therapeutic response to immune checkpoint inhibitors such as PD-1 or PD-L1 inhibitors is observed only in a small subset of cancer patients. The low mutational burden of "cold" tumors enables them to stay undetected from the host inflammatory response (innate resistance). Also, cancer cells develop a number of coping strategies in response to the selective pressure applied by the treatment with immune checkpoint inhibitors (recruitment of regulatory cells, defective antigen presentation, immunosuppressant mediators, reduced costimulation, and T cell apoptosis). There is thus a need for improved methods and compositions for the treatment of cancer that address the problem of resistances and lack of responsiveness to immune checkpoint inhibitors, particularly to PD(L)1 inhibitors. Anti-cancer activity of iadademstat is known from WO 2018/083138.

### SUMMARY OF THE INVENTION

The invention is based on the unexpected finding that the combination of iadademstat with immune checkpoint inhibitors, particularly PD(L)1 inhibitors (as defined herein), exhibits outstanding activity in inhibiting the growth of cancer cells as compared to treatment with the immune checkpoint inhibitor alone. Iadademstat can thus be used to sensitize tumors to PD(L)1 inhibitors and improve the responsiveness of tumors to PD(L)1 inhibitor therapy.

Thus, the instant invention relates to combinations of iadademstat, or a pharmaceutically acceptable salt or solvate thereof, with PD(L)1 inhibitors (as defined in the appended claims).

Accordingly, the present invention provides a combination comprising iadademstat, or a pharmaceutically acceptable salt or solvate thereof, and a PD(L)1 inhibitor as defined in the appended claims.

The present invention further provides a combination comprising iadademstat, or a pharmaceutically acceptable salt or solvate thereof, and a PD(L)1 inhibitor as defined in the appended claims for use in the treatment of cancer

Further aspects of the invention are disclosed in the appended claims.

### BRIEF DESCRIPTION OF THE DRAWINGS

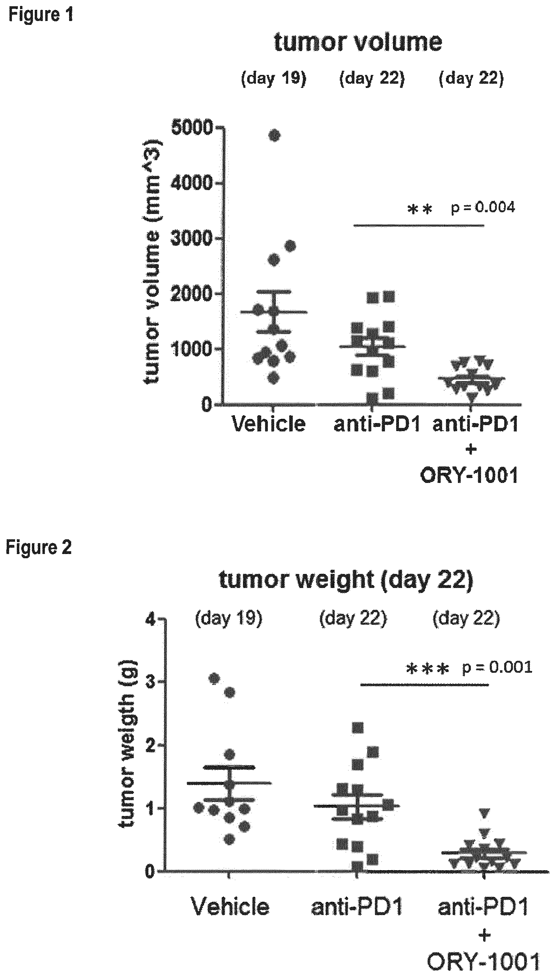

Figure 1 shows the effect of treatment with a combination of iadademstat (referred to as "ORY-1001") and a PD(L)1 inhibitor (triangles) in comparison with treatment with the PD(L)1 inhibitor alone (squares) on tumor volume in the mice B16F10 melanoma model, as explained in more detail in Example 1. Data is represented as mean± standard error of the mean (SEM); p=0.004.
Figure 2 shows the effect of treatment with a combination of iadademstat ("ORY-1001") and a PD(L)1 inhibitor (triangles) in comparison with treatment with the PD(L)1 inhibitor alone (squares) on tumor weight in the same mice B16F10 melanoma model, as explained in more detail in Example 1. Data is represented as mean± standard error of the mean (SEM); p=0.001.

### DETAILED DESCRIPTION OF THE INVENTION

The invention is defined by the claims. Any subject-matter falling outside the scope of the claims is provided for information purposes only. References herein to the methods of treatment by therapy are to be interpreted as references to compounds, combinations, pharmaceutical compositions and medicaments described herein for use in such methods.

As indicated above, the present invention is based on the discovery that the combination of iadademstat with PD(L)1 inhibitors exhibits outstanding anticancer activity, with superior anticancer efficacy as compared to treatment with the PD(L)1 inhibitor alone. The combined treatment with iadademstat and a PD(L)1 inhibitor causes a significant increase in the antitumor effect as compared to treatment with the PD(L)1 inhibitor alone, as illustrated in Example 1 and in Figures 1 and 2 using an *in vivo* murine melanoma model. Treatment with the combination produces statistically significant reductions in both the tumor volume and tumor weight compared to treatment with the PD(L)1 inhibitor alone, as shown in Figures 1 and 2, respectively. Thus, treament with iadademstat can enhance the efficacy of, or otherwise act synergistically with, PD(L)1 inhibitors.

Iadademstat can thus be used in combination with PD(L)1 inhibitors to treat cancer, including to increase the responsiveness of cancers to PD(L)1 inhibitor therapy and/or to sensitize refractory, non-responsive or relapsed cancers to PD(L)1 inhibitors.

In accordance with the present disclosure, a "PD(L)1 inhibitor" means a compound that inhibits the interaction of PD-1 with any of its ligands, PD-L1, PD-L2, or PD-L1 and PD-L2, inhibits PD-1 signaling, or reduces the PD-1 dependent inhibition of T cell-mediated immune responses against tumor cells. In accordance with the present invention, a PD(L)1 inhibitor includes a PD-1 inhibitor and a PD-L1 inhibitor. Examples thereof are provided below under the heading "PD(L)1 inhibitors".

In detail, the present invention provides a combination comprising iadademstat, or a pharmaceutically acceptable salt or solvate thereof, and a PD(L)1 inhibitor.

The present invention further provides a combination comprising iadademstat, or a pharmaceutically acceptable salt or solvate thereof, and a PD(L)1 inhibitor for use as a therapeutically active substance.

The present invention further provides a combination comprising iadademstat, or a pharmaceutically acceptable salt or solvate thereof, and a PD(L)1 inhibitor for use in therapy.

The present invention further provides a combination comprising iadademstat, or a pharmaceutically acceptable salt or solvate thereof, and a PD(L)1 inhibitor for use in the treatment of a disease.

The present invention further provides a combination comprising iadademstat, or a pharmaceutically acceptable salt or solvate thereof, and a PD(L)1 inhibitor for use in the treatment of cancer.

The present invention further provides iadademstat, or a pharmaceutically acceptable salt or solvate thereof, for use in the treatment of cancer in combination with a PD(L)1 inhibitor.

The present invention further provides a PD(L)1 inhibitor for use in the treatment of cancer in combination with iadademstat or a pharmaceutically acceptable salt or solvate thereof.

The present invention further provides iadademstat, or a pharmaceutically acceptable salt or solvate thereof, for use in the treatment of cancer, wherein the iadademstat or the pharmaceutically acceptable salt or solvate thereof is to be administered in combination with a PD(L)1 inhibitor.

The present invention further provides a PD(L)1 inhibitor for use in the treatment of cancer, wherein the PD(L)1 inhibitor is to be administered in combination with iadademstat or a pharmaceutically acceptable salt or solvate thereof.

The present invention further provides a method for treating cancer in a patient in need thereof, comprising administering to the patient a therapeutically effective amount of a combination comprising iadademstat, or a pharmaceutically acceptable salt or solvate thereof, and a PD(L)1 inhibitor.

The present invention further provides a method for treating cancer in a patient in need thereof, comprising administering to the patient a therapeutically effective amount of iadademstat, or a pharmaceutically acceptable salt or solvate thereof, and a therapeutically effective amount of a PD(L)1 inhibitor.

The present invention further provides the use of a combination comprising iadademstat, or a pharmaceutically acceptable salt or solvate thereof, and a PD(L)1 inhibitor for the treatment of cancer.

The present invention further provides the use of iadademstat, or a pharmaceutically acceptable salt or solvate thereof, for the treatment of cancer in combination with a PD(L)1 inhibitor.

The present invention further provides the use of a PD(L)1 inhibitor for the treatment of cancer in combination with iadademstat or a pharmaceutically acceptable salt or solvate thereof.

The invention further provides iadademstat or a pharmaceutically acceptable salt or solvate thereof for use in the treatment of cancer by sensitizing the cancer to treatment with a PD(L)1 inhibitor.

The invention further provides the use of iadademstat or a pharmaceutically acceptable salt or solvate thereof for the treatment of cancer by sensitizing the cancer to treatment with a PD(L)1 inhibitor.

The present invention further provides a method for treating cancer in a patient in need thereof by sensitizing the cancer to treatment with a PD(L)1 inhibitor, comprising administering to the patient a therapeutically effective amount of iadademstat, or a pharmaceutically acceptable salt or solvate thereof.

The invention further provides iadademstat or a pharmaceutically acceptable salt or solvate thereof for use in sensitizing cancer to treatment with a PD(L)1 inhibitor.

The invention further provides the use of iadademstat or a pharmaceutically acceptable salt or solvate thereof for sensitizing cancer to treatment with a PD(L)1 inhibitor.

The present invention further provides a method for sensitizing cancer to treatment with a PD(L)1 inhibitor in a patient in need thereof, comprising administering to the patient a therapeutically effective amount of iadademstat, or a pharmaceutically acceptable salt or solvate thereof.

The invention further provides iadademstat, or a pharmaceutically acceptable salt or solvate thereof, for use in increasing the responsiveness of a cancer to PD(L)1 inhibitor therapy.

The invention further provides a method for increasing the responsiveness of a cancer to PD(L)1 inhibitor therapy in a patient in need thereof, comprising administering to the patient a therapeutically effective amount of iadademstat or a pharmaceutically acceptable salt or solvate thereof.

The invention further provides iadademstat, or a pharmaceutically acceptable salt or solvate thereof, for use in sensitizing a refractory, non-responsive or relapsed cancer to PD(L)1 inhibitor therapy.

The invention further provides a method for sensitizing a refractory, non-responsive or relapsed cancer to PD(L)1 inhibitor therapy in a patient in need thereof, comprising administering to the patient a therapeutically effective amount of iadademstat or a pharmaceutically acceptable salt or solvate thereof.

The invention further provides iadademstat or a pharmaceutically acceptable salt or solvate thereof for use as an adjunct therapy to a PD(L)1 inhibitor.

The invention further provides the use of iadademstat or a pharmaceutically acceptable salt or solvate thereof as an adjunct therapy to a PD(L)1 inhibitor.

The invention further provides a method for the treatment of cancer in a patient in need thereof, which method comprises sensitizing said cancer through administration of iadademstat, or a pharmaceutically acceptable salt or solvate thereof, to said patient, followed by administering a therapeutically effective amount of a combination comprising iadademstat or a pharmaceutically acceptable salt or solvate thereof and a PD(L)1 inhibitor.

The invention further provides a method for the treatment of cancer in a patient in need thereof, which method comprises a first treatment where only iadademstat (or a pharmaceutically acceptable salt or solvate thereof) is administered to said patient (i.e. without a PD(L)1 inhibitor), prior to initiating the treatment with a combination comprising iadademstat (or a pharmaceutically acceptable salt or solvate thereof) and a PD(L)1 inhibitor.

In some embodiments, the cancer is a solid tumor.

In some embodiments, the cancer is suitable for treatment with a PD(L)1 inhibitor, wherein this is particularly a cancer for which a PD(L)1 inhibitor therapy is approved, i.e. that has received market approval by the regulatory authorities in at least one country.

In some embodiments, the cancer is selected from the group consisting of melanoma, small cell lung cancer (SCLC), non-small cell lung cancer (NSCLC), head and neck cancer, renal cell carcinoma, Hodgkin lymphoma, urothelial carcinoma, colorectal cancer, hepatocellular cancer, cutaneous squamous cell carcinoma, ovarian cancer, gastric cancer, gastroesophageal cancer, Merkel cell carcinoma, nasopharyngeal cancer, breast cancer (e.g. triple negative breast cancer) and esophageal squamous cell carcinoma.

In some embodiments, the cancer is selected from the group consisting of melanoma, small cell lung cancer (SCLC), non-small cell lung cancer (NSCLC), head and neck cancer, renal cell carcinoma, Hodgkin lymphoma, urothelial carcinoma, colorectal cancer, hepatocellular cancer, cutaneous squamous cell carcinoma, and Merkel cell carcinoma.

In some embodiments, the cancer is melanoma.

In some embodiments, the cancer is small cell lung cancer.

In some embodiments, the cancer is a cancer that is refractory, non-responsive or relapsed to PD(L)1 inhibitor therapy. Accordingly, in some embodiments, the cancer is a cancer that is refractory, non-responsive or relapsed to monotherapy with a PD(L)1 inhibitor.

In the methods and uses according to the invention, the patient is a human being or animal, preferably a human being.

In some embodiments, the patient having said cancer has received at least one prior treatment against said cancer comprising a PD(L)1 inhibitor (alone or in combination with other therapeutic agents).

### Iadademstat:

ladademstat is the International Non-proprietary Name (INN) for the compound of formula (I): 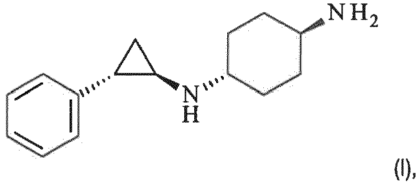 [CAS Reg. No. 1431304-21-0], which is also known as ORY-1001 or (trans)-N1-((1R,2S)-2-phenylcyclopropyl)cyclohexane-1,4-diamine. Iadademstat has been been described e.g. in Example 5 of International Patent Application WO 2013/057322. Pharmaceutically acceptable salts thereof are also described therein, including hydrochloride salts [CAS Reg. No. 1431303-72-8, dihydrochloride]. The most preferred pharmaceutically acceptable salt is a dihydrochloride salt. Iadademstat acts as a selective LSD1 inhibitor.

The terms "iadademstat", "Compound of formula (I)", (trans)-N1-((1R,2S)-2-phenylcyclopropyl)cyclohexane-1,4-diamine" and "ORY-1001" are used herein (i.e. throughout the present description and claims) interchangeably. Unless specifically indicated otherwise, any reference to iadademstat throughout the present description and claims includes iadademstat and any of its pharmaceutically acceptable salts or solvates. Preferably, iadademstat is used in the form of a pharmaceutically acceptable salt, preferably a hydrochloride salt, more preferably as di-hydrochloride salt.

Preferably, iadademstat (or a pharmaceutically acceptable salt or solvate thereof) is administered orally. Exemplary formulations which can be administered via peroral ingestion are described in more detail further below.

### PD(L)1 inhibitors:

As indicated earlier, as used herein a "PD(L)1 inhibitor" means a compound that inhibits the interaction of PD-1 with any of its ligands, PD-L1 and/or PD-L2, inhibits PD-1 signaling, or reduces the PD-1 dependent inhibition of T cell-mediated immune responses against tumor cells. Accordingly, the term "PD(L)1 inhibitor" includes PD-1 inhibitors, PD-L1 inhibitors and PD-L2 inhibitors. A PD(L)1 inhibitor according to the claimed invention may be a PD-1 inhibitor or a PD-L1 inhibitor.

PD(L)1 inhibitors are well known in the art, and any molecule acting as a PD(L)1 inhibitor can in principle be used in the context of the combinations, methods and uses according to the invention. The PD(L)1 inhibitor may be e.g. a small molecule, a peptide, an antibody or a vaccine.

In some embodiments, the PD(L)1 inhibitor is an antibody, more preferably a human antibody or humanized antibody.

Non-limiting examples of PD-1 inhibitors which can be used in accordance with the present invention include: Pembrolizumab (Merck & Co), Nivolumab (Bristol-Myers Squibb), Cemiplimab (also known as REGN-2810) (Regeneron Pharmaceuticals/Sanofi), Camrelizumab (also known as SHR-1210) (Shanghai Hengrui), Genolimzumab (also known as APL-501, GB226 or CBT-501) (Apollonomics Inc/Genor Biopharma), Tislelizumab (also known as BGB-A317) (Beigene/Celgene), Sintilimab (also known as IBI-308) (Eli Lilly/lnnovent), Toripalimab (also known as JS-001) (Shanghai Junshi), Spartalizumab (also known as PDR-001) (Novartis), AGEN-2034 (Agenus), AK-103 (Akeso Bio), AK-104 (Akeso Bio), AK-105 (Akeso Bio), AK-112 (Akeso Bio), AK-123 (Akeso Bio), AM-0001 (ARMO Bio/Eli Lilly), AMP-224 (Medimmune/GSK/NCI), AT16201 (AIMM), BCD-100 (Biocad), BH-2950 (Beijing Hanmi), BH-2996h (Beijing Hanmi), BI-754091 (Boehringer Ingelheim), BMS-1001 (Bristol-Myers Squibb), BMS-1166 (Bristol-Myers Squibb), CS-1003 (CStone Pharmaceuticals), CX-188 (CytomX), ENUM-244C8 (Enumeral), GLS-010 (Harbin/Wuxi/Arcus/Gloria Pharmaceuticals), hAb21 (Suzhou Stainwei), HLX-10 (Shanghai Henlius), IKT-202 (Icell Kealex), JNJ-63723283 (J&J), JTX-4014 (Jounce Therapeutics), KN0-46 (Alphamab), MEDI-0680 (also known as AMP-514) (Medimmune/GSK), MGA-012 (Macrogenics), MGD-013 (Macrogenics), PF-06801591 (Pfizer), PRS-332 (Pieris/Servier), RO7121661 (also known as RG-7769) (Roche), STI-A1110 (Servier/Sorrento), TSR-042 (Tesaro) and XmAb-20717 (Xencor).

Non-limiting examples of PD-L1 inhibitors which can be used in accordance with the present invention include: Atezolizumab, Avelumab, Durvalumab, AK-106 (Akeso Bio), APL-502 (also known as TQ-B2450) (Apollonomics Inc), AVA-004 (Avacta), BGB-A333 (BeiGene), BH-2996h (Beijing Hanmi), BMS-936559 (also known as MDX-11105) (Bristol-Myers Squibb), CA-170 (Curis/Aurigene), CA-327 (Curis/Aurigene), CBA-0710 (Sorrento), CK-301 (CheckPoint Therapeutics/TG Therapeutics), CS-1001 (CStone Pharmaceuticals), CX-072 (CytomX), FAZ-053 (Novartis), FS-118 (F Star/Merck KGaA), GR1405 (Genrix Biopharmaceutical), HLX-20 (Shanghai Henlius), IKT-201 (Icell Kealex), JS-003 (Shanghai Junshi), KD033 (Kadmon/Jinghua Pharma), KN-035 (3D Medicines Co, Ltd), KY-1003 (Kymab), LY3300054 (Eli Lilly), M-7824 (Merck KGaA), MCLA-145 (Merus/Incyte), MSB-2311 (Mabspace Biosciences), and SHR-1316 (also known as HTI-1088) (Atridia/Jiangsu Hengrui Therapeutics).

Preferred PD-1 inhibitors are Pembrolizumab, Nivolumab and Cemiplimab.

Pembrolizumab (also known as MK-3475 or lambrolizumab, Keytruda^{®}) was first approved by the Food and Drug Administration in 2014 for the treatment of melanoma. It was later approved for metastatic non-small cell lung cancer and head and neck squamous cell carcinoma. The drug has further been approved for SCLC, Hodgkin's lymphoma, primary mediastinal large B cell lymphoma, urothelial carcinoma, gastric cancer, esophageal cancer, cervical cancer, hepatocellular cancer, Merkel cell carcinoma, renal cell carcinoma and endometrial carcinoma.

Nivolumab (Opdivo^{®}) was developed by Bristol-Myers Squibb and first approved by the FDA in 2014 for the treatment of melanoma. It was later approved for non-small cell lung cancer, renal cell carcinoma, Hodgkin's lymphoma, head and neck squamous cell carcinoma, urothelial carcinoma, metastatic colon cancer and hepatocellular carcinoma, as well as SCLC.

Cemiplimab (Libtayo^{®}) was developed by Regeneron and first approved by the FDA in 2018 for the treatment of cutaneous squamous cell carcinoma (CSCC).

Preferred PD-L1 inhibitors are Atezolizumab, Avelumab and Durvalumab.

Atezolizumab (Tecentriq^{®}) was developed by Roche Genentech. In 2016, the FDA approved atezolizumab for urothelial carcinoma and non-small cell lung cancer. It has been later approved for SCLC and triple negative breast cancer.

Avelumab (Bavencio^{®}) was developed by Merck Serono and Pfizer. Avelumab was FDA approved in 2016 for the treatment of metastatic Merkel cell carcinoma, and has been later approved for urothelial carcinoma and renal cell carcinoma.

Durvalumab (Imfinzi^{®}) was developed by AstraZeneca. Durvalumab received first FDA approval in 2017 and is currently approved for the treatment of urothelial carcinoma and non-small cell lung cancer.

In some embodiments, the PD(L)1 inhibitor is a PD-1 inhibitor or a PD-L1 inhibitor.

In some embodiments, the PD(L)1 inhibitor is selected from the group consisting of Pembrolizumab, Nivolumab, Cemiplimab, Camrelizumab, Tislelizumab, Sintilimab, Toripalimab, Spartalizumab, AGEN-2034, AK-103, AK-104, AK-105, AK-112, AK-123, AM-0001, AMP-224, AT16201, BCD-100, BH-2950, BH-2996h, BI-754091, BMS-1001, BMS-1166, CS-1003, CX-188, ENUM-244C8, GLS-010, hAb21, HLX-10, IKT-202, JNJ-63723283, JTX-4014, KN0-46, MEDI-0680, MGA-012, MGD-013, PF-06801591, PRS-332, RO7121661, STI-A1110, TSR-042, XmAb-20717, Atezolizumab, Avelumab, Durvalumab, AK-106, APL-502, AVA-004, BGB-A333, BH-2996h, BMS-936559, CA-170, CA-327, CBA-0710, CK-301, CS-1001, CX-072, FAZ-053, FS-118, GR1405, HLX-20, IKT-201, JS-003, KD033, KN-035, KY-1003, LY3300054, M-7824, MCLA-145, MSB-2311, and SHR-1316. In some embodiments, the PD(L)1 inhibitor is selected from the group consisting of Pembrolizumab, Nivolumab, Cemiplimab, Atezolizumab, Avelumab and Durvalumab.

In some embodiments, the PD(L)1 inhibitor is a PD-1 inhibitor. In some embodiments, the PD-1 inhibitor is selected from the group consisting of Pembrolizumab, Nivolumab, Cemiplimab, Camrelizumab, Tislelizumab, Sintilimab, Toripalimab, Spartalizumab, AGEN-2034, AK-103, AK-104, AK-105, AK-112, AK-123, AM-0001, AMP-224, AT16201, BCD-100, BH-2950, BH-2996h, BI-754091, BMS-1001, BMS-1166, CS-1003, CX-188, ENUM-244C8, GLS-010, hAb21, HLX-10, IKT-202, JNJ-63723283, JTX-4014, KN0-46, MEDI-0680, MGA-012, MGD-013, PF-06801591, PRS-332, RO7121661, STI-A1110, TSR-042, and XmAb-20717. In some embodiments, the PD-1 inhibitor is selected from the group consisting of Pembrolizumab, Nivolumab and Cemiplimab.

In some embodiments, the PD(L)1 inhibitor is a PD-L1 inhibitor. In some embodiments, the PD-L1 inhibitor is selected from the group consisting of Atezolizumab, Avelumab, Durvalumab, AK-106, APL-502, AVA-004, BGB-A333, BH-2996h, BMS-936559, CA-170, CA-327, CBA-0710, CK-301, CS-1001, CX-072, FAZ-053, FS-118, GR1405, HLX-20, IKT-201, JS-003, KD033, KN-035, KY-1003, LY3300054, M-7824, MCLA-145, MSB-2311, and SHR-1316. In some embodiments, the PD-L1 inhibitor is selected from the group consisting of Atezolizumab, Avelumab and Durvalumab.

In some embodiments, the invention provides a combination comprising iadademstat, or a pharmaceutically acceptable salt or solvate thereof, and a PD(L)1 inhibitor selected from a PD-1 inhibitor and PD-L1 inhibitor. In some embodiments, the PD(L)1 inhibitor is selected from the group consisting of Pembrolizumab, Nivolumab, Cemiplimab, Camrelizumab, Tislelizumab, Sintilimab, Toripalimab, Spartalizumab, AGEN-2034, AK-103, AK-104, AK-105, AK-112, AK-123, AM-0001, AMP-224, AT16201, BCD-100, BH-2950, BH-2996h, BI-754091, BMS-1001, BMS-1166, CS-1003, CX-188, ENUM-244C8, GLS-010, hAb21, HLX-10, IKT-202, JNJ-63723283, JTX-4014, KN0-46, MEDI-0680, MGA-012, MGD-013, PF-06801591, PRS-332, RO7121661, STI-A1110, TSR-042, XmAb-20717, Atezolizumab, Avelumab, Durvalumab, AK-106, APL-502, AVA-004, BGB-A333, BH-2996h, BMS-936559, CA-170, CA-327, CBA-0710, CK-301, CS-1001, CX-072, FAZ-053, FS-118, GR1405, HLX-20, IKT-201, JS-003, KD033, KN-035, KY-1003, LY3300054, M-7824, MCLA-145, MSB-2311, and SHR-1316. In some embodiments, the PD(L)1 inhibitor is selected from the group consisting of Pembrolizumab, Nivolumab, Cemiplimab, Atezolizumab, Avelumab and Durvalumab.

In some embodiments, the invention provides a combination comprising iadademstat, or a pharmaceutically acceptable salt or solvate thereof, and a PD-1 inhibitor. In some embodiments, the PD-1 inhibitor is selected from the group consisting of Pembrolizumab, Nivolumab, Cemiplimab, Camrelizumab, Tislelizumab, Sintilimab, Toripalimab, Spartalizumab, AGEN-2034, AK-103, AK-104, AK-105, AK-112, AK-123, AM-0001, AMP-224, AT16201, BCD-100, BH-2950, BH-2996h, BI-754091, BMS-1001, BMS-1166, CS-1003, CX-188, ENUM-244C8, GLS-010, hAb21, HLX-10, IKT-202, JNJ-63723283, JTX-4014, KN0-46, MEDI-0680, MGA-012, MGD-013, PF-06801591, PRS-332, RO7121661, STI-A1110, TSR-042, and XmAb-20717. In some preferred embodiments, the PD-1 inhibitor is selected from the group consisting of Pembrolizumab, Nivolumab and Cemiplimab.

In some embodiments, the invention provides a combination comprising iadademstat, or a pharmaceutically acceptable salt or solvate thereof, and a PD-L1 inhibitor. In some preferred embodiments, the PD-L1 inhibitor is selected from the group consisting of Atezolizumab, Avelumab, Durvalumab, AK-106, APL-502, AVA-004, BGB-A333, BH-2996h, BMS-936559, CA-170, CA-327, CBA-0710, CK-301, CS-1001, CX-072, FAZ-053, FS-118, GR1405, HLX-20, IKT-201, JS-003, KD033, KN-035, KY-1003, LY3300054, M-7824, MCLA-145, MSB-2311, and SHR-1316. In some preferred embodiments, the PD-L1 inhibitor is selected from the group consisting of Atezolizumab, Avelumab and Durvalumab.

In some embodiments, the disclosure provides a combination comprising iadademstat, or a pharmaceutically acceptable salt or solvate thereof, and a PD-L2 inhibitor.

Another embodiment provides pharmaceutical compositions or medicaments comprising the combinations as described herein and a pharmaceutically acceptable excipient, as well as methods of using the compound of formula (I) to prepare such combinations, compositions and medicaments.

Any reference to iadademstat throughout this specification includes a reference to the compound as such, i.e. the corresponding compound in non-salt form (e.g., as a free base) or in the form of any pharmaceutically acceptable salt or solvate thereof, as well as a reference to any pharmaceutical composition comprising said compound and one or more pharmaceutically acceptable excipients or carriers.

Any reference to a PD(L)1 inhibitor throughout this specification includes a reference to the PD(L)1 inhibitor as such, or in the form of any pharmaceutically acceptable salt or solvate thereof (if applicable), as well as a reference to any pharmaceutical composition comprising said PD(L)1 inhibitor and one or more pharmaceutically acceptable excipients or carriers.

### Pharmaceutical Formulations

ladademstat and the PD(L)1 inhibitor for use in the combinations as described herein as well as the pharmaceutical compositions as described herein may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, or intralesional administration. Parenteral infusions include intramuscular, intravenous, intraarterial, intraperitoneal, or subcutaneous administration.

Iadademstat and the other therapeutic agent for use in the combinations as described herein as well as the pharmaceutical compositions as described herein may be administered in any convenient pharmaceutical product form, e.g., tablets, powders, capsules, solutions, dispersions, suspensions, syrups, sprays, suppositories, gels, emulsions, patches, etc. Such compositions may comprise components conventional in pharmaceutical preparations, e.g., diluents, carriers, pH modifiers, preservatives, solubilizers, stabilizers, wetting agents, emulsifiers, sweeteners, colorants, flavorants, salts for varying the osmotic pressure, buffers, masking agents, antioxidants, and further active agents. They can also comprise still other therapeutically valuable substances.

A typical formulation is prepared by mixing iadademstat or the other therapeutic agent as described herein or a combination as described herein and a pharmaceutically acceptable excipient. Suitable excipients are well known to those skilled in the art and are described in detail in, e.g. Ansel's Pharmaceutical Dosage Forms and Drug Delivery Systems (2004) Lippincott, Williams & Wilkins, Philadelphia; Remington: The Science and Practice of Pharmacy (2000) Lippincott, Williams & Wilkins, Philadelphia; and Handbook of Pharmaceutical Excipients (2005) Pharmaceutical Press, Chicago The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

For oral delivery, the compound can be incorporated into a formulation that includes pharmaceutically acceptable carriers such as binders (e.g., gelatin, cellulose, gum tragacanth), excipients (e.g., starch, lactose), lubricants (e.g., magnesium stearate, silicon dioxide), disintegrating agents (e.g., alginate, Primogel, and corn starch), and sweetening or flavoring agents (e.g., glucose, sucrose, saccharin, methyl salicylate, and peppermint). The formulation can be orally delivered, e.g., in the form of enclosed gelatin capsules or compressed tablets. Capsules and tablets can be prepared by any conventional techniques. The capsules and tablets can also be coated with various coatings known in the art to modify the flavors, tastes, colors, and shapes of the capsules and tablets. In addition, liquid carriers such as fatty oil can also be included in capsules. Suitable oral formulations can also be in the form of suspension, syrup, chewing gum, wafer, elixir, and the like. If desired, conventional agents for modifying flavors, tastes, colors, and shapes of the special forms can also be included. In addition, for convenient administration by enteral feeding tube in patients unable to swallow, the active compounds can be dissolved in an acceptable lipophilic vegetable oil vehicle such as olive oil, corn oil and safflower oil.

The compound can also be administered parenterally in the form of solution or suspension, or in lyophilized form capable of conversion into a solution or suspension form before use. In such formulations, diluents or pharmaceutically acceptable carriers such as sterile water and physiological saline buffer can be used. Other conventional solvents, pH buffers, stabilizers, anti-bacteria agents, surfactants, and antioxidants can all be included. For example, useful components include sodium chloride, acetates, citrates or phosphates buffers, glycerin, dextrose, fixed oils, methyl parabens, polyethylene glycol, propylene glycol, sodium bisulfate, benzyl alcohol, ascorbic acid, and the like. The parenteral formulations can be stored in any conventional containers such as vials and ampoules.

For topical administration, the compound can be formulated into lotions, creams, ointments, gels, powders, pastes, sprays, suspensions, drops and aerosols. Thus, one or more thickening agents, humectants, and stabilizing agents can be included in the formulations. Examples of such agents include, but are not limited to, polyethylene glycol, sorbitol, xanthan gum, petrolatum, beeswax, or mineral oil, lanolin, squalene, and the like.

A special form of topical administration is delivery by a transdermal patch. Methods for preparing transdermal patches are disclosed, e.g., in Brown, et al. (1988) Ann. Rev. Med. 39:221-229.

Subcutaneous implantation for sustained release of the compound may also be a suitable route of administration. This entails surgical procedures for implanting an active compound in any suitable formulation into a subcutaneous space, *e.g.,* beneath the anterior abdominal wall. See, e.g., Wilson et al. (1984) J. Clin. Psych. 45:242-247. Hydrogels can be used as a carrier for the sustained release of active compounds. Hydrogels are generally known in the art. They are typically made by crosslinking high molecular weight biocompatible polymers into a network, which swells in water to form a gel like material. Preferably, hydrogels are biodegradable or biosorbable. For purposes of this invention, hydrogels made of polyethylene glycols, collagen, or poly(glycolic-co-L-lactic acid) may be useful. See, e.g., Phillips et al. (1984) J. Pharmaceut. Sci., 73: 1718-1720.

The pharmaceutical compositions, like oral and parenteral compositions, can be formulated in unit dosage forms for ease of administration and uniformity of dosage. As used herein, "unit dosage forms" refers to physically discrete units suitable as unitary dosages for administration to subjects, each unit containing a predetermined quantity of active ingredient calculated to produce the desired therapeutic effect, in association with one or more suitable pharmaceutical carriers.

In therapeutic applications, pharmaceutical compositions are to be administered in a manner appropriate to the disease to be treated, as determined by a person skilled in the medical arts. An appropriate dose and suitable duration and frequency of administration can vary within wide limits and will be determined by such factors as the condition of the patient, the type and severity of the disease, the particular form of the active ingredient(s), the method of administration, among others. In general, an appropriate dose and administration regimen provides the pharmaceutical composition in an amount sufficient to provide therapeutic benefit, for example an improved clinical outcome, such as more frequent complete or partial remissions, or longer disease-free and/or overall survival, or lessening of symptoms severity, or any other objetively identifiable improvement as noted by the clinician. Effective doses may generally be assessed or extrapolated using experimental models like dose-response curves derived from in vitro or animal model test systems, or from clinical trials.

Suitable doses for a PD(L)-1 inhibitor are typically those presently used by the physician for the respective immune checkpoint inhibitor, in particular the dose(s) approved by the respective govemmental authorities. Other doses may also be possible, for example the dose of PD(L)-1 inhibitor may be lowered due to the combined action of the newly identified combinations of said PD(L)-1 inhibitors with iadademstat.

The combinations as described herein may be administered as a simultaneous or sequential regimen. When administered sequentially, the combination may be administered in two or more administrations. The combined administration includes coadministration, using separate formulation, and consecutive administration in either order, wherein preferably there is a time period while both (or all) active agents simultaneously exert their biological activities. The combinations of the invention may also be administered as a single pharmaceutical composition comprising the compound of formula (I) and the PD(L)1 inhibitor.

The pharmaceutical compositions of the invention can be included in a container, pack or dispenser together with instructions for administration.

In another embodiment of the invention, an article of manufacture, or "kit", containing a combination useful for the treatment of the diseases and disorders described above is provided.

In some embodiments, the article of manufacture or kit comprises a container and a combination according to the invention as described herein.

In some embodiments, the article of manufacture or kit comprises: a) a container comprising iadademstat (or a pharmaceutically acceptable salt or solvate thereof), and b) a container comprising a PD(L)1 inhibitor.

The articles of manufacture or kits may further comprise a label or package insert. The term "package insert" is used to refer to instructions customarily included in commercial packages of therapeutic products, that contain information about the indications, usage, dosage, administration, contraindications and/or warnings concerning the use of such therapeutic products. Suitable containers include, for example, bottles, vials, syringes, blister pack, etc. The container may be formed from a variety of materials such as glass or plastic. The container may hold a combination, or a formulation thereof, which is effective for treating the condition and may have a sterile access port (for example, the container may be an intravenous solution bag or a vial having a stopper pierceable by a hypodermic injection needle). The label or package insert indicates that the composition is used for treating the condition of choice, such as cancer, e.g a cancer as described herein. In one embodiment, the label or package inserts indicates that the composition comprising the combination can be used to treat cancer. Alternatively, or additionally, the article of manufacture may further comprise a further container comprising a pharmaceutically acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The kit may further comprise directions for the administration of the combination, and, if present, the second pharmaceutical formulation. For example, if the kit comprises a first composition comprising iadademstat, or a pharmaceutically acceptable salt thereof, and a second pharmaceutical composition comprising a PD(L)1 inhibitor, the kit may further comprise directions for the simultaneous, sequential or separate administration of the first and second pharmaceutical compositions to a patient in need thereof.

In another embodiment, the kits are suitable for the delivery of solid oral forms of a combination, such as tablets or capsules. Such a kit preferably includes a number of unit dosages. Such kits can include a card having the dosages oriented in the order of their intended use. An example of such a kit is a "blister pack". Blister packs are well known in the packaging industry and are widely used for packaging pharmaceutical unit dosage forms. If desired, a memory aid can be provided, for example in the form of numbers, letters, or other markings or with a calendar insert, designating the days in the treatment schedule in which the dosages can be administered.

According to one embodiment, a kit may comprise (a) a first container with iadademstat, or a pharmaceutically acceptable salt or solvate thereof contained therein; (b) a second container with a PD(L)1 inhibitor. Alternatively, or additionally, the kit may comprise another container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Where the kit comprises a composition of iadademstat, or a pharmaceutically acceptable salt or solvate thereof and a PD(L)1 inhibitor, the kit may comprise a container for containing the separate compositions such as a divided bottle or a divided foil packet, however, the separate compositions may also be contained within a single, undivided container. Typically, the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

### Definitions

Unless defined otherwise, all technical and scientific terms used herein have the same meaning as commonly understood by one of ordinary skill in the art to which this invention pertains.

The following definitions apply throughout the present specification and claims, unless specifically indicated otherwise.

A "patient" or "subject" for the purposes of the present invention includes both humans and other animals, particularly mammals. Thus, the methods and uses of the invention are applicable to both human therapy and veterinary applications. In a preferred aspect the subject or patient is a mammal, and in the most preferred aspect the subject or patient is a human (e.g. a male or female human).

The terms "treatment", "treating" and the like are used herein to generally mean obtaining a desired pharmacological and/or physiological effect. The effect may be prophylactic in terms of completely or partially preventing a disease (herein, cancer) or symptom thereof and/or may be therapeutic in terms of partially or completely curing or ameliorating a disease (i.e. cancer) and/or a symptom or adverse effect attributed to the disease or partially or completely halting the progression of a disease and/or a symptom or adverse effect attributed to the disease. The term "treatment" as used herein covers any treatment of a disease (i.e. cancer) in a patient and includes, without limitation, any one or more of the following: (a) preventing cancer in a patient which may be predisposed/at risk of developing cancer; (b) delaying the onset of cancer; (c) inhibiting cancer, i.e. arresting, delaying or slowing down its development/progression; or (d) relieving the cancer, i.e. causing (complete or partial) regression, correction or alleviation of cancer. The present invention specifically and distinctly relates to each one of these forms of treatment.

As used herein, the term "therapeutically effective amount" refers to the amount sufficient to produce a desired biological effect (e.g., a therapeutic effect) in a subject. Accordingly, a therapeutically effective amount of a compound may be an amount which is sufficient to treat a disease (i.e. cancer), and/or delay the onset or progression of the disease, and/or alleviate one or more symptoms of the disease, when administered to a subject suffering from or susceptible to that disease.

As used herein, a "pharmaceutically acceptable salt" is intended to mean a salt that retains the biological effectiveness of the free acids and/or bases of the specified compound and that is not biologically or otherwise undesirable. A compound may possess a sufficiently acidic, a sufficiently basic, or both functional groups, and accordingly react with any of a number of inorganic or organic bases, and inorganic and organic acids, to form a pharmaceutically acceptable salt. Exemplary pharmaceutically acceptable salts include those salts prepared by reaction of a compound according to the invention, e.g. iadademstat, with a mineral or organic acid, such as hydrochlorides, hydrobromides, sulfates, pyrosulfates, bisulfates, sulfites, bisulfites, phosphates, monohydrophosphates, dihydrophosphates, metaphosphates, pyrophosphates, chlorides, bromides, iodides, nitrates, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, gamma-hydroxybutyrates, glycollates, tartrates, methane-sulfonates, ethane-sulfonates, propanesulfonates, benzenesulfonates, toluenesulfonates, trifluoromethansulfonates, naphthalene-1-sulfonates, naphthalene-2-sulfonates, mandelates, pyruvates, stearates, ascorbates, or salicylates. When a compound carries an acidic moiety, suitable pharmaceutically acceptable salts thereof may include alkali metal salts, e.g. sodium or potassium salts; alkaline earth metal salts, e.g. calcium or magnesium salts; and salts formed with suitable organic ligands such as ammonia, alkylamines, hydroxyalkylamines, lysine, arginine, N-methylglucamine, procaine and the like. Pharmaceutically acceptable salts are well known in the art.

As used herein, a "pharmaceutically acceptable solvate" refers to a complex of variable stoichiometry formed by a solute and a pharmaceutically acceptable solvent such as water, ethanol and the like. A complex with water is known as a hydrate. It is to be understood that the invention encompasses pharmaceutically acceptable solvates of iadademstat in non-salt form and also in the form of a pharmaceutically acceptable salt thereof.

As used herein, a "pharmaceutically acceptable carrier" or "pharmaceutically acceptable excipient" refers to non-API (API refers to Active Pharmaceutical Ingredient) substances such as disintegrators, binders, fillers, and lubricants used in formulating pharmaceutical products. They are generally safe for administering to humans according to established governmental standards, including those promulgated by the United States Food and Drug Administration and/or the European Medicines Agency. Pharmaceutically acceptable carriers or excipients are well known to those skilled in the art.

As used herein, a "small molecule" refers to an organic compound with a molecular weight below 900 daltons, preferably below 500 daltons. The molecular weight is the mass of a molecule and is calculated as the sum of the atomic weights of each constituent element multiplied by the number of atoms of that element in the molecular formula.

The term "antibody" according to the invention is used in its broadest sense and comprises all antibodies, antibody fragments, and derivatives thereof that are capable of binding to an antigen, in this case the immune checkpoint. This encompasses the complete monoclonal antibodies and also the epitope-binding fragments of these antibodies. In this connection, the epitope binding fragments (also referred to herein as antibody fragments or antibody derivatives) comprise all regions of the antibody that are capable of binding to the antigen. Examples of particular antibody fragments in accordance with the invention comprise, but are not limited to, Fab, Fab', F(ab')2, Fd, individual chain (single chain) variable fragments (scFv), single-chain antibodies, disulfide-linked variable fragments (sdFv), and fragments that either contain a variable region of the light chain (VL) or a variable region of the heavy chain (VH). Moreover, they include recombinantly prepared antibodies, such as diabodies, and tetrabodies. Antibody fragments contain the variable regions either alone or in combination with further regions that are selected from the hinge region and the first, second and third regions of the constant region (CH1, CH2, CH3). Also, the term antibody comprises chimeric antibodies in which different regions of the antibody originate from different species, for example, antibodies with a murine variable region combined with a human constant region. Antibody fragments are optionally linked with each other by a linker. The linker comprises a short (particularly 10 to 20 amino acid residues), flexible peptide sequence that is selected such that the antibody fragment has such a three dimensional folding of VL and VH that it exhibits the antigen specificity of the complete antibody. Moreover, particular linkers are comprised of a peptide sequence that can increase the protease resistance of the antibody derivatives.

The term "inhibitor" as used herein denotes a compound which competes with, decreases, blocks, inhibits, abrogates or interferes in any way with the binding of a particular ligand to a particular receptor or enzyme and/or which decreases, blocks, inhibits, abrogates or interferes in any way with the activity of a particular protein, e.g. of a receptor or enzyme.

As used herein, the term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

As used herein, unless explicitly indicated otherwise or contradicted by context, the terms "a", "an" and "the" are used interchangeably with "one or more" and "at least one". Thus, for example, a composition comprising "a" PD(L)1 inhibitor can be interpreted as referring to a composition comprising "one or more" PD(L)1 inhibitors.

### EXAMPLES

The following examples are provided for illustration of the invention. They should not be considered as limiting the scope of the invention, but merely as being representative thereof. Results are also presented and described in the Figures and Figure legends.

### Example 1: Evaluation of the effect of iadademstat in combination with a PD(L)1 inhibitor in an in vivo model of melanoma in mice

### Method:

B16F10 melanoma cells were maintained in vitro as a monolayer culture in DMEM-High glucose (Sigma, D5796) supplemented with 10% fetal bovine serum (ThermoFisher, 10500064). Cells in an exponential growth were harvested for tumor inoculation. 6-8 weeks C57/BL6 female mice were inoculated subcutaneously in the right flank with B16F10 melanoma cells (0.5 x 10⁶) in 0.050 mL of DMEM (Dulbecco's Modified Eagle Medium):matrigel 1:1 (15 mice per group). Mice from groups #1 and #2 received vehicle-treated cells, whereas B16F10 cells injected on group #3 were exposed for 96h to 5 nM iadademstat 2HCl, immediately before inoculation. Treatment started on the day of inoculation. Animals were injected i.p. with either sterile Dulbecco's phosphate-buffered saline (DPBS) (group #1) or anti-PD1 mAb (100 µg of clone RMP1-14 from BioXcell; cat. # BE0146; rat anti-mouse mAb) on days 4, 7 and 11 (groups #2 and #3). Animals from group #3 received in addition iadademstat 2HCl 10 µg/Kg by oral gavage, starting from day 0 until termination of the experiment (day 22) following a 5/2 administration schedule (1111100). The concentration of iadademstat for *in vitro* treatment is expressed as free base, while the dose administered *in vivo* refers to the iadademstat dihydrochloride salt. Tumor volumes were measured twice weekly in two dimensions using a caliper, and the volume was expressed in mm³ using the formula: V = 0.5 a × b² where "a" and "b" are the long and short diameters of the tumor, respectively. Tumor weight was measured at study termination. Statistical analysis was performed using unpaired t-test.

### Results:

A statistically significant (p=0.004) difference in tumor volumes (TV) was observed comparing animals treated with anti-PD1 mAb as single agent (average TV = 1099 mm³ on day 22) or with the combination iadademstat + anti-PD1 mAb (average TV = 509 mm³ on day 22). Average tumor volume in vehicle controls was 1677 mm³ on day 19.

The measured tumor weights also showed a statistically significant reduction in tumor weight (p=0.001) in the animals treated with the combination iadademstat + anti-PD1 mAb compared to the animals receiving anti-PD1 mAb alone, with an average weight of 0.29 g in tumors treated with the combination iadademstat + anti-PD1 mAb (collected on day 22), compared to an average tumor weight of 1.03 g and 1.39 g in the animals receiving treatment with anti-PD1 mAb as single agent (collected on day 22) or vehicle (collected on day 19), respectively.

Using similar methods to the one described in Example 1, the therapeutic effects of combinations of iadademstat with PD(L)1 inhibitors in other cancer types can be verified.

## Claims

1. A combination comprising iadademstat, or a pharmaceutically acceptable salt or solvate thereof, and a PD(L)1 inhibitor, wherein the PD(L)1 inhibitor is a PD-1 inhibitor or a PD-L1 inhibitor.

2. A combination comprising iadademstat, or a pharmaceutically acceptable salt or solvate thereof, and a PD(L)1 inhibitor for use in therapy, wherein the PD(L)1 inhibitor is a PD-1 inhibitor or a PD-L1 inhibitor.

3. A combination comprising iadademstat, or a pharmaceutically acceptable salt or solvate thereof, and a PD(L)1 inhibitor for use in the treatment of a disease, wherein the PD(L)1 inhibitor is a PD-1 inhibitor or a PD-L1 inhibitor.

4. A combination comprising iadademstat, or a pharmaceutically acceptable salt or solvate thereof, and a PD(L)1 inhibitor for use in the treatment of cancer, wherein the PD(L)1 inhibitor is a PD-1 inhibitor or a PD-L1 inhibitor.

5. ladademstat, or a pharmaceutically acceptable salt or solvate thereof, for use in the treatment of cancer in combination with a PD(L)1 inhibitor, wherein the PD(L)1 inhibitor is a PD-1 inhibitor or a PD-L1 inhibitor.

6. A PD(L)1 inhibitor, which is a PD-1 inhibitor or a PD-L1 inhibitor, for use in the treatment of cancer in combination with iadademstat or a pharmaceutically acceptable salt or solvate thereof.

7. The combination according to claim 1, the combination for use according to any one of claims 2 to 4, or the compound for use according to claim 5 or 6, wherein:
(a) the PD(L)1 inhibitor is selected from the group consisting of Pembrolizumab, Nivolumab, Cemiplimab, Camrelizumab, Tislelizumab, Sintilimab, Toripalimab, Spartalizumab, AGEN-2034, AK-103, AK-104, AK-105, AK-112, AK-123, AM-0001, AMP-224, AT16201, BCD-100, BH-2950, BH-2996h, BI-754091, BMS-1001, BMS-1166, CS-1003, CX-188, ENUM-244C8, GLS-010, hAb21, HLX-10, IKT-202, JNJ-63723283, JTX-4014, KN0-46, MEDI-0680, MGA-012, MGD-013, PF-06801591, PRS-332, RO7121661, STI-A1110, TSR-042, XmAb-20717, Atezolizumab, Avelumab, Durvalumab, AK-106, APL-502, AVA-004, BGB-A333, BH-2996h, BMS-936559, CA-170, CA-327, CBA-0710, CK-301, CS-1001, CX-072, FAZ-053, FS-118, GR1405, HLX-20, IKT-201, JS-003, KD033, KN-035, KY-1003, LY3300054, M-7824, MCLA-145, MSB-2311, and SHR-1316, or
(b) the PD(L)1 inhibitor is selected from the group consisting of Pembrolizumab, Nivolumab, Cemiplimab, Atezolizumab, Avelumab and Durvalumab; or
(c) the PD(L)1 inhibitor is a PD-1 inhibitor; wherein preferably the PD-1 inhibitor is selected from the group consisting of Pembrolizumab, Nivolumab, Cemiplimab, Camrelizumab, Tislelizumab, Sintilimab, Toripalimab, Spartalizumab, AGEN-2034, AK-103, AK-104, AK-105, AK-112, AK-123, AM-0001, AMP-224, AT16201, BCD-100, BH-2950, BH-2996h, BI-754091, BMS-1001, BMS-1166, CS-1003, CX-188, ENUM-244C8, GLS-010, hAb21, HLX-10, IKT-202, JNJ-63723283, JTX-4014, KN0-46, MEDI-0680, MGA-012, MGD-013, PF-06801591, PRS-332, RO7121661, STI-A1110, TSR-042, and XmAb-20717, or
(d) the PD(L)1 inhibitor is a PD-L1 inhibitor, wherein preferably the PD-L1 inhibitor is selected from the group consisting of Atezolizumab, Avelumab, Durvalumab, AK-106, APL-502, AVA-004, BGB-A333, BH-2996h, BMS-936559, CA-170, CA-327, CBA-0710, CK-301, CS-1001, CX-072, FAZ-053, FS-118, GR1405, HLX-20, IKT-201, JS-003, KD033, KN-035, KY-1003, LY3300054, M-7824, MCLA-145, MSB-2311, and SHR-1316.

8. The combination according to claim 1, the combination for use according to any one of claims 2 to 4, or the compound for use according to claim 5 or 6, wherein the PD(L)1 inhibitor is a PD-1 inhibitor selected from the group consisting of Pembrolizumab, Nivolumab and Cemiplimab.

9. The combination according to claim 1, the combination for use according to any one of claims 2 to 4, or the compound for use according to claim 5 or 6, wherein the PD(L)1 inhibitor is a PD-L1 inhibitor selected from the group consisting of Atezolizumab, Avelumab and Durvalumab.

10. The combination for use according to any one of claims 4 or 7 to 9 or the compound for use according to any one of claims 5 to 9, wherein:
(a) the cancer is selected from the group consisting of melanoma, small cell lung cancer, non-small cell lung cancer (NSCLC), head and neck cancer, renal cell carcinoma, Hodgkin lymphoma, urothelial carcinoma, colorectal cancer, hepatocellular cancer, cutaneous squamous cell carcinoma, ovarian cancer, gastric cancer, gastroesophageal cancer, Merkel cell carcinoma, nasopharyngeal cancer, breast cancer, and esophageal squamous cell carcinoma; or
(b) the cancer is melanoma.

11. The combination for use according to any one of claims 4 or 7 to 9 or the compound for use according to any one of claims 5 to 9, wherein the cancer is small cell lung cancer.

12. The combination for use according to any one of claims 4 or 7 to 11 or the compound for use according to any one of claims 5 to 11, wherein the cancer is a cancer that is refractory, non-responsive or relapsed to PD-1 inhibitor or PD-L1 inhibitor therapy.

13. The combination according to any one of claims 1 or 7 to 9, the combination for use according to any one of claims 2 to 4 or 7 to 12, or the compound for use according to any one of claims 5 to 12, wherein iadademstat or a pharmaceutically acceptable salt or solvate thereof is iadademstat dihydrochloride.

14. The combination for use according to any one of claims 2 to 4 or 7 to 13 or the compound for use according to any one of claims 5 to 13, wherein iadademstat or the pharmaceutically acceptable salt or solvate thereof is administered orally.

15. The combination for use according to any one of claims 2 to 4 or 7 to 14 or the compound for use according to any one of claims 5 to 14, wherein iadademstat or the pharmaceutically acceptable salt or solvate thereof and the PD(L)1 inhibitor are administered using separate formulations.

16. The combination for use according to any one of claims 2 to 4 or 7 to 15 or the compound for use according to any one of claims 5 to 15, wherein iadademstat or the pharmaceutically acceptable salt or solvate thereof and the PD(L)1 inhibitor are administered as a simultaneous regimen.

17. The combination for use according to any one of claims 2 to 4 or 7 to 15 or the compound for use according to any one of claims 5 to 15, wherein iadademstat or the pharmaceutically acceptable salt or solvate thereof and the PD(L)1 inhibitor are administered as a sequential regimen.

## Patentansprüche

1. Eine Kombination, umfassend ladademstat oder ein pharmazeutisch verträgliches Salz oder Solvat davon und einen PD(L)1-Inhibitor, wobei der PD(L)1-Inhibitor ein PD-1-Inhibitor oder ein PD-L1-Inhibitor ist.

2. Eine Kombination, umfassend ladademstat oder ein pharmazeutisch verträgliches Salz oder Solvat davon und einen PD(L)1-Inhibitor zur Verwendung in der Therapie, wobei der PD(L)1-Inhibitor ein PD-1-Inhibitor oder ein PD-L1-Inhibitor ist.

3. Eine Kombination, umfassend ladademstat oder ein pharmazeutisch verträgliches Salz oder Solvat davon und einen PD(L)1-Inhibitor zur Verwendung bei der Behandlung einer Erkrankung, wobei der PD(L)1-Inhibitor ein PD-1-Inhibitor oder ein PD-L1-Inhibitor ist.

4. Eine Kombination, umfassend ladademstat oder ein pharmazeutisch verträgliches Salz oder Solvat davon und einen PD(L)1-Inhibitor zur Verwendung bei der Behandlung von Krebs, wobei der PD(L)1-Inhibitor ein PD-1-Inhibitor oder ein PD-L1-Inhibitor ist.

5. ladademstat oder ein pharmazeutisch verträgliches Salz oder Solvat davon zur Verwendung bei der Behandlung von Krebs in Kombination mit einem PD(L)1-Inhibitor, wobei der PD(L)1-Inhibitor ein PD-1-Inhibitor oder ein PD-L1-Inhibitor ist.

6. Ein PD(L)1-Inhibitor, der ein PD-1-Inhibitor oder ein PD-L1-Inhibitor ist, zur Verwendung bei der Behandlung von Krebs in Kombination mit ladademstat oder einem pharmazeutisch verträglichen Salz oder Solvat davon.

7. Die Kombination nach Anspruch 1, die Kombination zur Verwendung nach einem der Ansprüche 2 bis 4 oder die Verbindung zur Verwendung nach Anspruch 5 oder 6, wobei:
(a) der PD(L)1-Inhibitor ausgewählt ist aus der Gruppe bestehend aus Pembrolizumab, Nivolumab, Cemiplimab, Camrelizumab, Tislelizumab, Sintilimab, Toripalimab, Spartalizumab, AGEN-2034, AK-103, AK-104, AK-105, AK-112, AK-123, AM-0001, AMP-224, AT16201, BCD-100, BH-2950, BH-2996h, BI-754091, BMS-1001, BMS-1166, CS-1003, CX-188, ENUM-244C8, GLS-010, hAb21, HLX-10, IKT-202, JNJ-63723283, JTX-4014, KN0-46, MEDI-0680, MGA-012, MGD-013, PF-06801591, PRS-332, RO7121661, STI-A1110, TSR-042, XmAb-20717, Atezolizumab, Avelumab, Durvalumab, AK-106, APL-502, AVA-004, BGB-A333, BH-2996h, BMS-936559, CA-170, CA-327, CBA-0710, CK-301, CS-1001, CX-072, FAZ-053, FS-118, GR1405, HLX-20, IKT-201, JS-003, KD033, KN-035, KY-1003, LY3300054, M-7824, MCLA-145, MSB-2311 und SHR-1316, oder
(b) der PD(L)1-Inhibitor ausgewählt ist aus der Gruppe bestehend aus Pembrolizumab, Nivolumab, Cemiplimab, Atezolizumab, Avelumab und Durvalumab; oder
(c) der PD(L)1-Inhibitor ein PD-1-Inhibitor ist; wobei der PD-1-Inhibitor vorzugsweise ausgewählt ist aus der Gruppe bestehend aus Pembrolizumab, Nivolumab, Cemiplimab, Camrelizumab, Tislelizumab, Sintilimab, Toripalimab, Spartalizumab, AGEN-2034, AK-103, AK-104, AK-105, AK-112, AK-123, AM-0001, AMP-224, AT16201, BCD-100, BH-2950, BH-2996h, BI-754091, BMS-1001, BMS-1166, CS-1003, CX-188, ENUM-244C8, GLS-010, hAb21, HLX-10, IKT-202, JNJ-63723283, JTX-4014, KN0-46, MEDI-0680, MGA-012, MGD-013, PF-06801591, PRS-332, RO7121661, STI-A1110, TSR-042 und XmAb-20717; oder
(d) der PD(L)1-Inhibitor ein PD-L1-Inhibitor ist, wobei der PD-L1-Inhibitor vorzugsweise ausgewählt ist aus der Gruppe bestehend aus Atezolizumab, Avelumab, Durvalumab, AK-106, APL-502, AVA-004, BGB-A333, BH-2996h, BMS-936559, CA-170, CA-327, CBA-0710, CK-301, CS-1001, CX-072, FAZ-053, FS-118, GR1405, HLX-20, IKT-201, JS-003, KD033, KN-035, KY-1003, LY3300054, M-7824, MCLA-145, MSB-2311 und SHR-1316.

8. Die Kombination nach Anspruch 1, die Kombination zur Verwendung nach einem der Ansprüche 2 bis 4 oder die Verbindung zur Verwendung nach Anspruch 5 oder 6, wobei der PD(L)1-Inhibitor ein PD-1-Inhibitor, ausgewählt aus der Gruppe bestehend aus Pembrolizumab, Nivolumab und Cemiplimab, ist.

9. Die Kombination nach Anspruch 1, die Kombination zur Verwendung nach einem der Ansprüche 2 bis 4 oder die Verbindung zur Verwendung nach Anspruch 5 oder 6, wobei der PD(L)1-Inhibitor ein PD-L1-Inhibitor, ausgewählt aus der Gruppe bestehend aus Atezolizumab, Avelumab und Durvalumab, ist.

10. Die Kombination zur Verwendung nach einem der Ansprüche 4 oder 7 bis 9 oder die Verbindung zur Verwendung nach einem der Ansprüche 5 bis 9, wobei:
(a) der Krebs ausgewählt ist aus der Gruppe bestehend aus Melanom, kleinzelligem Lungenkrebs, nicht-kleinzelligem Lungenkrebs (NSCLC), Kopf-Hals-Krebs, Nierenzellkarzinom, Hodgkin-Lymphom, Urothelkarzinom, Kolorektalkrebs, Leberzellkarzinom, kutanem Plattenepithelkarzinom, Eierstockkrebs, Magenkrebs, gastroösophagealem Krebs, Merkelzellkarzinom, Nasopharynxkarzinom, Brustkrebs und Plattenepithelkarzinom der Speiseröhre; oder
(b) der Krebs ein Melanom ist.

11. Die Kombination zur Verwendung nach einem der Ansprüche 4 oder 7 bis 9 oder die Verbindung zur Verwendung nach einem der Ansprüche 5 bis 9, wobei der Krebs kleinzelliger Lungenkrebs ist.

12. Die Kombination zur Verwendung nach einem der Ansprüche 4 oder 7 bis 11 oder die Verbindung zur Verwendung nach einem der Ansprüche 5 bis 11, wobei der Krebs ein Krebs ist, der gegenüber einer PD-1-Inhibitor- oder PD-L1-Inhibitor-Therapie refraktär ist, nicht anspricht oder rezidivierend ist.

13. Die Kombination nach einem der Ansprüche 1 oder 7 bis 9, die Kombination zur Verwendung nach einem der Ansprüche 2 bis 4 oder 7 bis 12 oder die Verbindung zur Verwendung nach einem der Ansprüche 5 bis 12, wobei ladademstat oder ein pharmazeutisch verträgliches Salz oder Solvat davon ladademstat-Dihydrochlorid ist.

14. Die Kombination zur Verwendung nach einem der Ansprüche 2 bis 4 oder 7 bis 13 oder die Verbindung zur Verwendung nach einem der Ansprüche 5 bis 13, wobei ladademstat oder das pharmazeutisch verträgliche Salz oder Solvat davon oral verabreicht wird.

15. Die Kombination zur Verwendung nach einem der Ansprüche 2 bis 4 oder 7 bis 14 oder die Verbindung zur Verwendung nach einem der Ansprüche 5 bis 14, wobei ladademstat oder das pharmazeutisch verträgliche Salz oder Solvat davon und der PD(L)1-Inhibitor unter Verwendung getrennter Formulierungen verabreicht werden.

16. Die Kombination zur Verwendung nach einem der Ansprüche 2 bis 4 oder 7 bis 15 oder die Verbindung zur Verwendung nach einem der Ansprüche 5 bis 15, wobei ladademstat oder ein pharmazeutisch verträgliches Salz oder Solvat davon und der PD(L)1-Inhibitor als gleichzeitiges Therapieschema verabreicht werden.

17. Die Kombination zur Verwendung nach einem der Ansprüche 2 bis 4 oder 7 bis 15 oder die Verbindung zur Verwendung nach einem der Ansprüche 5 bis 15, wobei ladademstat oder das pharmazeutisch verträgliche Salz oder Solvat und der PD(L)1-Inhibitor als sequentielles Therapieschema verabreicht werden.

## Revendications

1. Combinaison comprenant de l'iadademstat, ou un sel ou solvate pharmaceutiquement acceptable de celui-ci, et un inhibiteur de PD(L)1, dans laquelle l'inhibiteur de PD(L)1 est un inhibiteur de PD-1 ou un inhibiteur de PD-L1.

2. Combinaison comprenant de l'iadademstat, ou un sel ou solvate pharmaceutiquement acceptable de celui-ci, et un inhibiteur de PD(L)1 pour une utilisation en thérapie, dans laquelle l'inhibiteur de PD(L)1 est un inhibiteur de PD-1 ou un inhibiteur de PD-L1.

3. Combinaison comprenant de l'iadademstat, ou un sel ou solvate pharmaceutiquement acceptable de celui-ci, et un inhibiteur de PD(L)1 pour une utilisation dans le traitement d'une maladie, dans laquelle l'inhibiteur de PD(L)1 est un inhibiteur de PD-1 ou un inhibiteur de PD-L1.

4. Combinaison comprenant de l'iadademstat, ou un sel ou solvate pharmaceutiquement acceptable de celui-ci, et un inhibiteur de PD(L)1 pour une utilisation dans le traitement du cancer, dans laquelle l'inhibiteur de PD(L)1 est un inhibiteur de PD-1 ou un inhibiteur de PD-L1.

5. Iadademstat, ou sel ou solvate pharmaceutiquement acceptable de celui-ci, pour une utilisation dans le traitement du cancer en combinaison avec un inhibiteur de PD(L)1, dans lequel l'inhibiteur de PD(L)1 est un inhibiteur de PD-1 ou un inhibiteur de PD-L1.

6. Inhibiteur de PD(L)1, qui est un inhibiteur de PD-1 ou un inhibiteur de PD-L1, pour une utilisation dans le traitement du cancer en combinaison avec l'iadademstat ou un sel ou solvate pharmaceutiquement acceptable de celui-ci.

7. Combinaison selon la revendication 1, combinaison pour l'utilisation selon l'une quelconque des revendications 2 à 4, ou composé pour l'utilisation selon la revendication 5 ou 6, dans laquelle ou lequel :
(a) l'inhibiteur de PD(L)1 est choisi dans le groupe consistant en Pembrolizumab, Nivolumab, Cemiplimab, Camrelizumab, Tislelizumab, Sintilimab, Toripalimab, Spartalizumab, AGEN-2034, AK-103, AK-104, AK-105, AK-112, AK-123, AM-0001, AMP-224, AT16201, BCD-100, BH-2950, BH-2996h, BI-754091, BMS-1001, BMS-1166, CS-1003, CX-188, ENUM-244C8, GLS-010, hAb21, HLX-10, IKT-202, JNJ-63723283, JTX-4014, KN0-46, MEDI-0680, MGA-012, MGD-013, PF-06801591, PRS-332, RO7121661, STI-A1110, TSR-042, XmAb-20717, Atézolizumab, Avélumab, Durvalumab, AK-106, APL-502, AVA-004, BGB-A333, BH-2996h, BMS-936559, CA-170, CA-327, CBA-0710, CK-301, CS-1001, CX-072, FAZ-053, FS-118, GR1405, HLX-20, IKT-201, JS-003, KD033, KN-035, KY-1003, LY3300054, M-7824, MCLA-145, MSB-2311, et SHR-1316, ou
(b) l'inhibiteur de PD(L)1 est choisi dans le groupe consistant en Pembrolizumab, Nivolumab, Cemiplimab, Atézolizumab, Avélumab et Durvalumab ; ou
(c) l'inhibiteur de PD(L)1 est un inhibiteur de PD-1 ; où de préférence l'inhibiteur de PD-1 est choisi dans le groupe consistant en Pembrolizumab, Nivolumab, Cemiplimab, Camrelizumab, Tislelizumab, Sintilimab, Toripalimab, Spartalizumab, AGEN-2034, AK-103, AK-104, AK-105, AK-112, AK-123, AM-0001, AMP-224, AT16201, BCD-100, BH-2950, BH-2996h, BI-754091, BMS-1001, BMS-1166, CS-1003, CX-188, ENUM-244C8, GLS-010, hAb21, HLX-10, IKT-202, JNJ-63723283, JTX-4014, KN0-46, MEDI-0680, MGA-012, MGD-013, PF-06801591, PRS-332, RO7121661, STI-A1110, TSR-042, et XmAb-20717 ;
ou
(d) l'inhibiteur de PD(L)1 est un inhibiteur de PD-L1 ; où de préférence l'inhibiteur de PD-L1 est choisi dans le groupe consistant en Atézolizumab, Avélumab, Durvalumab, AK-106, APL-502, AVA-004, BGB-A333, BH-2996h, BMS-936559, CA-170, CA-327, CBA-0710, CK-301, CS-1001, CX-072, FAZ-053, FS-118, GR1405, HLX-20, IKT-201, JS-003, KD033, KN-035, KY-1003, LY3300054, M-7824, MCLA-145, MSB-2311, et SHR-1316.

8. Combinaison selon la revendication 1, combinaison pour l'utilisation selon l'une quelconque des revendications 2 à 4, ou composé pour l'utilisation selon la revendication 5 ou 6, dans laquelle ou lequel l'inhibiteur de PD(L)1 est un inhibiteur de PD-1 choisi dans le groupe consistant en Pembrolizumab, Nivolumab et Cemiplimab.

9. Combinaison selon la revendication 1, combinaison pour l'utilisation selon l'une quelconque des revendications 2 à 4, ou composé pour l'utilisation selon la revendication 5 ou 6, dans laquelle ou lequel l'inhibiteur de PD(L)1 est un inhibiteur de PD-L1 choisi dans le groupe consistant en Atézolizumab, Avélumab et Durvalumab.

10. Combinaison pour l'utilisation selon l'une quelconque des revendications 4 ou 7 à 9, ou composé pour l'utilisation selon l'une quelconque des revendications 5 à 9, dans laquelle ou lequel :
(a) le cancer est choisi dans le groupe consistant en mélanome, cancer du poumon à petites cellules, cancer du poumon non à petites cellules (CPNPC), cancer de la tête et du cou, carcinome des cellules rénales, lymphome hodgkinien, carcinome urothélial, cancer colorectal, carcinome hépatocellulaire, carcinome épidermoïde cutané, cancer des ovaires, cancer gastrique, cancer gastro-œsophagien, carcinome des cellules de Merkel, cancer nasopharyngé, cancer du sein et carcinome épidermoïde œsophagien ; ou
(b) le cancer est un mélanome.

11. Combinaison pour l'utilisation selon l'une quelconque des revendications 4 ou 7 à 9, ou composé pour l'utilisation selon l'une quelconque des revendications 5 à 9, dans laquelle ou lequel le cancer est le cancer du poumon à petites cellules.

12. Combinaison pour l'utilisation selon l'une quelconque des revendications 4 ou 7 à 11, ou composé pour l'utilisation selon l'une quelconque des revendications 5 à 11, dans laquelle ou lequel le cancer est un cancer qui est réfractaire, non répondant ou récidivant à la thérapie par inhibiteur de PD-1 ou inhibiteur de PD-L1.

13. Combinaison pour l'utilisation selon l'une quelconque des revendications 1 ou 7 à 9, combinaison pour l'utilisation selon l'une quelconque des revendications 2 à 4 ou 7 à 12, ou composé pour l'utilisation selon l'une quelconque des revendications 5 à 12, dans laquelle ou lequel l'iadademstat ou un sel ou solvate pharmaceutiquement acceptable de celui-ci est le chlorhydrate d'iadademstat.

14. Combinaison pour l'utilisation selon l'une quelconque des revendications 2 à 4 ou 7 à 13, ou composé pour l'utilisation selon l'une quelconque des revendications 5 à 13, dans laquelle ou lequel l'iadademstat ou le sel ou solvate pharmaceutiquement acceptable de celui-ci est administré par voie orale.

15. Combinaison pour l'utilisation selon l'une quelconque des revendications 2 à 4 ou 7 à 14, ou composé pour l'utilisation selon l'une quelconque des revendications 5 à 14, dans laquelle ou lequel l'iadademstat ou le sel ou solvate pharmaceutiquement acceptable de celui-ci et l'inhibiteur de PD(L)1 sont administrés en utilisant des formulations séparées.

16. Combinaison pour l'utilisation selon l'une quelconque des revendications 2 à 4 ou 7 à 15, ou composé pour l'utilisation selon l'une quelconque des revendications 5 à 15, dans laquelle ou lequel l'iadademstat ou le sel ou solvate pharmaceutiquement acceptable de celui-ci et l'inhibiteur de PD(L)1 sont administrés sous forme de posologie simultanée.

17. Combinaison pour l'utilisation selon l'une quelconque des revendications 2 à 4 ou 7 à 15, ou composé pour l'utilisation selon l'une quelconque des revendications 5 à 15, dans laquelle ou lequel l'iadademstat ou le sel ou solvate pharmaceutiquement acceptable de celui-ci et l'inhibiteur de PD(L)1 sont administrés sous forme de posologie séquentielle.
